# EUROPEAN PATENT APPLICATION

(11) **EP 4 070 793 A1**
(43) Date of publication of application: **12.10.2022**
(21) Application number: 20896662.2
(22) Date of filing: 20.11.2020
(51) Int. Cl.: A61K 31/428, A61K 31/165, A61P 25/16

(54) **PHARMACEUTICAL COMPOSITION, COMPLEMENTARY KIT AND APPLICATION THEREOF**

(30) Priority: 06.12.2019 CN 201911239173
(71) Applicant: Shanghai Pharmaceuticals Holding Co., Ltd., Shanghai 201203 (CN)
(72) Inventor: SU, Wei, Shanghai 201203 (CN); XIA, Guangxin, Shanghai 201203 (CN); WANG, Xuesong, Shanghai 201203 (CN); KE, Ying, Shanghai 201203 (CN); BI, Guangqing, Shanghai 201203 (CN); HUANG, Jian, Shanghai 201203 (CN)
(74) Representative: Dehns
(86) International application number: PCT/CN2020/130345
(87) International publication number: WO 2021/109880

(57) **Abstract**

A pharmaceutical composition, a complementary kit and an application thereof; the pharmaceutical composition comprises pramipexole and safinamide, the mass ratio of pramipexole to safinamide being 1:300-1:30; and the pharmaceutical composition can be used to prepare drugs for treating Parkinson's disease, has a good curative effect and has few side effects, and can more effectively improve the condition of a patient. The capabilities thereof in restoring dopamine levels are better than those from using pramipexole or safinamide alone. In addition, the composition has better neuroprotective activity, and therefore damage to the dopaminergic system and the normal sensitivity of the dopamine system to dopamine agonists are reduced. The described composition can restore or balance a potential unbalanced response to "increasing dopaminergic treatment" that is caused by the use of a dopamine agonist alone; insofar as the same or a better therapeutic effect is achieved, the composition can reduce the amount of pramipexole when used alone so as to reduce the risk of side effects and drug resistance.

## Description

The present application claims priority to Chinese Patent Application No. 2019112391738 filed on December 06, 2019, which is incorporated herein by reference in their entireties.

### TECHNICAL FIELD

The present disclosure relates to a pharmaceutical composition, a kit of parts and use thereof.

### BACKGROUND

Parkinson's disease is a lifelong disease that requires continuous treatment, and medication remains the leading medical intervention. The main directions for symptomatic treatment of Parkinson's disease are to increase dopamine synthesis, stimulate dopamine receptor activity, inhibit reuptake of dopamine by presynaptic receptors and inhibit dopamine catabolism.

Dopamine receptor agonists are a group of compounds that are functionally similar to dopamine, but chemically different. It can activate dopamine receptors like dopamine, thus playing a similar role to dopamine. Pramipexole is a compound which is a common dopamine agonist used in clinical applications. However, it may cause side effects such as impulse-control disorders, peripheral edema, psychosis and sedation, which greatly restrict the clinical use of the compound.

Monoamine oxidase B (MAO-B) is one of the key enzymes for dopamine catabolism in brain, and inhibition of monoamine oxidase B activity can reduce the degradation of dopamine and increase dopamine concentration in brain. Representatives of monoamine oxidase B (MAO-B) inhibitors are selegiline, rasagiline and safinamide.

Pharma Two B company found that the combination of two drugs with complementary mechanisms (pramipexole and rasagiline) have better efficacy in treating Parkinson's disease than either monotherapy of higher doses (see Patent No. WO2009147681). Also, a combination containing a dopamine agonist of a lower dose is more beneficial and has minimal side effects than a dopamine agonist monotherapy of a higher dose, while achieving similar therapeutic effects.

Rasagiline, however, is irreversible in inhibiting MAO-B. Although high levels of monoamine oxidase are associated with neurological disorders, monoamine oxidase is also an essential metabolic enzyme in the body. As such, long-term irreversible inhibition of monoamine oxidase has certain effects on cardiac function and cardiovascular system. This leads to a condition in which some patients experienced cardiac discomfort after receiving rasagiline. Therefore, those skilled in the art have been working on the development of a treatment that is more advantageous to the patients, featuring efficacy in treating Parkinson's disease and minimized side effect.

Safinamide is a third-generation monoamine oxidase B (MAO-B) inhibitor, and is a novel, highly efficient, reversible and highly specific MAO-B inhibitor. It demonstrates a 5000-fold inhibitory strength to MAO-B than that to MAO-A, which is 127 folds for selegiline and 103 folds for rasagiline. The inhibitory effect of the compound on MAO-B is reversible, and the physiological function can be completely restored 8 h after discontinuation. It was reported that in a 24-week, randomized, double-blind, placebo-controlled, parallel, multicenter study (Stocchi F, Borgohain R, Onofrj M, Schapira AH, Bhatt M, Lucini V, et al. A randomized, double-blind, placebo-controlled trial of safinamide as add-on therapy in early Parkinson's disease patients. Mov Disord Off J Mov Disord Soc. 2012;27(1):106-12), 269 patients with early PD who had received a fixed dose of dopamine (DA) receptor agonist were randomized to a placebo group (n = 90), a safinamide 100 mg/d group (n = 90) and a safinamide 200 mg/d group (n = 89), with the primary efficacy endpoint being total UPDRS score (movement function). It was found that a high dose of safinamide (200 mg/d) in combination with DA receptor agonist has no effect in improving movement symptoms, whereas a low dose (100 mg/d) was effective in the improvement of movement symptoms. However, it is not clear in the art what DA receptor agonist the safinamide is used in combination with, and what the ratio is, such that a combination synergism can be achieved.

### SUMMARY

The present disclosure is intended to overcome the defects of significant side effect, poor efficacy and the like of a pharmaceutical composition in treating Parkinson's disease in the prior art, and provides a pharmaceutical composition, a kit of parts and use thereof. The pharmaceutical composition disclosed herein comprises pramipexole and safinamide in a certain ratio, can be used in preparing a medicament for treating Parkinson's disease, has good efficacy and few side effects, and can more effectively improve the condition of a patient. The pharmaceutical composition disclosed herein has better ability to restore dopamine levels than pramipexole or safinamide alone, and a better neuroprotective activity, thus reducing the impairment of the dopaminergic system and the normal sensitivity of the dopamine system to dopamine agonists. The composition can restore or equilibrate the potential inequilibrium response to the dopaminergic increase treatment caused by dopamine agonist alone; the composition disclosed herein reduces the amount of pramipexole when used alone to reduce the risks of side effects and drug resistance, while achieving the same or better therapeutic effect.

The inventors have conducted extensive studies on drugs for treating Parkinson's disease and surprisingly found that when safinamide is combined with pramipexole in a specific ratio, the condition of patients can be more effectively improved.

In order to solve the above technical problems, the present disclosure provides, in a first aspect, a pharmaceutical composition comprising pramipexole and safinamide, wherein a mass ratio of pramipexole and safinamide is 1:300 to 1:30.

Preferably, a mass ratio of pramipexole and safinamide is 1:200 to 1:50, preferably 150 to 1:75, for example, 1:50, 1:55, 1:60, 1:65, 1:70, 1:75, 1:80, 1:85, 1:90, 1:95, 1:100, 1:110, 1:120, 1:130, 1:140, 1:150, 1:160, 1:170, 1:180 or 1:190. In the present disclosure, the mass ration of pramipexole and safinamide may also be, for example, 1:300 to 1:75, 1:300 to 1:100, 1:300 to 1:150, 1:150 to 1:30, 1:150 to 1:75, 1:150 to 1:100, 1:100 to 1:30, 1:100 to 1:75, 1:75 to 1:30 or the like.

Preferably, an amount of pramipexole is 0.01 to 5 mg.

Preferably, an amount of safinamide is 0.3 to 1500 mg.

Preferably, an amount of pramipexole is 0.05 to 0.5 mg.

Preferably, an amount of safinamide is 7.5 to 100 mg.

In the present disclosure, an amount of pramipexole may be, for example, 0.02, 0.04, 0.05, 0.06, 0.08, 0.1, 0.15, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 3 or 4 mg, for example, 0.1, 0.15 or 0.2 mg.

In the present disclosure, an amount of safinamide may be, for example, 0.4, 0.5, 0.6, 0.7, 0.8, 0.9, 1, 1.1, 1.2, 1.3, 1.4, 1.5, 1.6, 1.7, 1.8, 1.9, 2, 3, 4, 5, 6, 7, 7.5, 8, 9, 10, 12, 14, 15, 16, 18, 20, 30, 40, 50, 60, 70, 80, 90, 100, 110, 120, 130, 140, 150, 200, 300, 400, 500, 600, 700, 800, 900, 1000, 1100, 1200, 1300 or 1400 mg, preferably 10, 15, 20 or 50 mg.

Preferably, the pharmaceutical composition comprises 0.05 mg of pramipexole and 7.5 mg of safinamide, 0.05 mg of pramipexole and 10 mg of safinamide, 0.05 mg of pramipexole and 15 mg of safinamide, 0.1 mg of pramipexole and 15 mg of safinamide, 0.15 mg of pramipexole and 15 mg of safinamide, 0.2 mg of pramipexole and 15 mg of safinamide, 0.5 mg of pramipexole and 15 mg of safinamide, 0.6 mg of pramipexole and 60 mg of safinamide, 1.2 mg of pramipexole and 120 mg of safinamide, 0.3 mg of pramipexole and 30 mg of safinamide, or 0.2 mg of pramipexole and 20 mg of safinamide.

More preferably, the pharmaceutical composition further comprises at least one pharmaceutically acceptable carrier.

In order to solve the above technical problems, the present disclosure, in a second aspect, provides a kit of parts comprising a kit A and a kit B, wherein the kit A comprises pramipexole and the kit B comprises safinamide, or the kit A comprises pramipexole and safinamide and the kit B comprises another drug; wherein a mass ratio of pramipexole and safinamide is1:300 to 1:30, preferably 150 to 1:75, more preferably 1:200 to 1:50, for example, 1:50, 1:55, 1:60, 1:65, 1:70, 1:75, 1:80, 1:85, 1:90, 1:95, 1:100, 1:110, 1:120, 1:130, 1:140, 1:150, 1:160, 1:170, 1:180 or 1:190. In the present disclosure, a mass ratio of pramipexole and safinamide may also be, for example, 1:300 to 1:75, 1:300 to 1:100, 1:300 to 1:150, 1:150 to 1:30, 1:150 to 1:75, 1:150 to 1:100, 1:100 to 1:30, 1:100 to 1:75, 1:75 to 1:30 or the like.

Preferably, an amount of pramipexole is 0.01 to 5 mg, more preferably 0.05 to 0.5 mg.

Preferably, an amount of safinamide is 0.3 to 1500 mg, more preferably 7.5 to 100 mg.

In the present disclosure, an amount of pramipexole may be, for example, 0.02, 0.04, 0.05, 0.06, 0.08, 0.1, 0.15, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 3 or 4 mg, for example, 0.1, 0.15 or 0.2 mg.

In the present disclosure, an amount of safinamide may be, for example, 0.4, 0.5, 0.6, 0.7, 0.8, 0.9, 1.0, 1.1, 1.2, 1.3, 1.4, 1.5, 1.6, 1.7, 1.8, 1.9, 2, 3, 4, 5, 6, 7, 7.5, 8, 9, 10, 12, 14, 15, 16, 18, 20, 30, 40, 50, 60, 70, 80, 90, 100, 110, 120, 130, 140, 150, 200, 300, 400, 500, 600, 700, 800, 900, 1000, 1100, 1200, 1300 or 1400 mg, preferably 10, 15, 20 or 50 mg.

Preferably, the kit of parts comprises 0.05 mg of pramipexole and 7.5 mg of safinamide, 0.05 mg of pramipexole and 10 mg of safinamide, 0.05 mg of pramipexole and 15 mg of safinamide, 0.1 mg of pramipexole and 15 mg of safinamide, 0.15 mg of pramipexole and 15 mg of safinamide, 0.2 mg of pramipexole and 15 mg of safinamide, 0.5 mg of pramipexole and 15 mg of safinamide, 0.6 mg of pramipexole and 60 mg of safinamide, 1.2 mg of pramipexole and 120 mg of safinamide, 0.3 mg of pramipexole and 30 mg of safinamide, or 0.2 mg of pramipexole and 20 mg of safinamide.

In order to solve the above technical problems, the present disclosure, in a third aspect, provides use of the pharmaceutical composition according to any the first aspect or the kit of parts according to the second aspect of the present disclosure in preparing a medicament for treating Parkinson's disease, for improving movement function and/or for increasing dopamine concentration.

In order to solve the above technical problems, the present disclosure further provides use of the pharmaceutical composition according to any the first aspect or the kit of parts according to the second aspect of the present disclosure in treating Parkinson's disease, in improving movement function and/or in increasing dopamine concentration.

In order to solve the above technical problems, the present disclosure, in a fourth aspect, provides a method of treating Parkinson's disease comprising: administering to a patient in need thereof the pharmaceutical composition according to the first aspect or the kit of parts according to the second aspect of the present disclosure.

In the present disclosure, the ratio of the two components, pramipexole and safinamide, is generally precisely calibrated and the two components are preferably formulated in a single dosage form designed for optimal pharmacokinetic performance, efficacy and patient adherence. The term "fixed dose combination" as used herein describes a single dose formulation containing a precise ratio (i.e., a fixed dose) of the two different drugs. For treatments based on a variety of drugs, the precise ratio of the components, timing, administration and pharmacokinetic aspects play extremely important roles. In order to determine the optimal fixed dose combination, not only the combination/synergistic effect and potency are important, but also the relative pharmacokinetics of each component and the optimal dosage form.

In the pharmaceutical composition, both pramipexole and safinamide can be released in a manner of immediate release and/or controlled release; for example, controlled-release pramipexole and safinamide, controlled-release pramipexole and immediate-release safinamide, controlled- and immediate-release pramipexole and controlled- and immediate-release safinamide, wherein up to 50% by mass of pramipexole and safinamide are in a controlled-release form.

The pharmaceutical composition disclosed herein may be in any suitable dosage form, such as tablets, gels and bilayer tablets. The tablet may be a matrix tablet, wherein the release of the active ingredient is controlled by diffusion of the active ingredient through the gel formed by expansion of a hydrophilic polymer upon contact with a dissolving liquid (*in vitro*) or an enteric liquid (*in vivo*). Many polymers capable of forming such gels have been described in the prior art, for example cellulose derivatives, in particular cellulose ethers, such as hydroxypropyl cellulose, hydroxymethyl cellulose, methyl cellulose or methylhydroxypropyl cellulose, and among the different commercial grades of these ethers are those which exhibit a rather high viscosity. The tablets may also be bilayer tablets consisting of two or more different granulation layers pressed together by laying each layer on the top of another, each individual layer containing a different active ingredient. The bilayer tablet has a sandwich appearance in that the edges of each layer or region are exposed.

The superior therapeutic effect of the combination disclosed herein over today's monotherapy and combination therapy of Parkinson's disease results from unique ratios and/or formulations.

Although expressed in mg/kg in animal experiments, clinical dosages are generally expressed in mg, and thus the pharmaceutical compositions, kits of parts, use and methods disclosed herein for treating Parkinson's disease are generally expressed in mg, without excluding the meaning that it can represent mg/kg.

When the composition disclosed herein is administered to a patient, safinamide can be administered initially in a dose of 50 mg, PO, once every day at the same time period; after two weeks, the dose may be increased to 100 mg once every day, depending on individual needs and tolerability.

The above preferred conditions may be combined arbitrarily to obtain preferred embodiments of the present disclosure without departing from the general knowledge in the art.

The reagents and starting materials used in the present disclosure are commercially available.

The present disclosure has the following positive and advantageous effects: The pharmaceutical composition disclosed herein comprises pramipexole and safinamide in a certain ratio, can be used in preparing a medicament for treating Parkinson's disease, has good efficacy and few side effects, and can more effectively improve the condition of a patient. The pharmaceutical composition disclosed herein has better ability to restore dopamine levels than pramipexole or safinamide alone, and imparts neuroprotective activity, thus reducing the impairment of the dopaminergic system and the normal sensitivity of the dopamine system to dopamine agonists. The composition can restore or equilibrate the potential inequilibrium response to the dopaminergic increase treatment caused by dopamine agonist alone, and can reduce the amount of pramipexole to reduce the risks of side effects and drug resistance, while achieving the same or better therapeutic effect.

### BRIEF DESCRIPTION OF THE DRAWINGS

FIG. 1 illustrates the average dopamine concentration in mouse brain obtained by measuring the amount of mouse striatal dopamine in Example 1.
FIG. 2 illustrates the trend of APO-induced circling behavior in 6-OHDA-induced rat PD model.
FIG. 3 illustrates the revolutions of APO-induced circling in 6-OHDA-induced rat PD model on Day 29 post-dose.

### DETAILED DESCRIPTION

The present disclosure is further illustrated by the following examples, which are not intended to limit the present disclosure. Experimental procedures without specified conditions in the following examples were conducted in accordance with conventional procedures and conditions, or in accordance with the manufacturer's manual.

### Example 1. In vivo characterization of the combination in an MPTP (1-methyl-4-phenyl-1,2,3,6-tetrahydropyridine)-induced mouse Parkinson's disease model

### (1) Materials

Animals: male C57BL/6J mice (SPF grade), from Nanjing Biomedical Research Institute of Nanjing University, qualification number: 201900586. The mice were fed in a closed environment at 20 to 26 °C and a relative humidity of 40% to 70% in a 14/10-h light/dark cycle, and were provided with free access to water and food.

### Compounds:

Pramipexole (compound A), batch No.: BPL20171102, molecular weight: 302.26, purity: 99.93%, Shanghai Boyier Chemical Co., Ltd

Safinamide (compound B), batch No.: G059-S-2-151102, molecular weight: 398.45, purity: 99.97%, manufactured by Shanghai Pharmaceuticals Holding Co., Ltd.

MPTP, batch No.: N1012A, molecular weight: 209.71, purity: 98% (for induction of Parkinson's disease model), purchased from Meilunbio.

### (2) Methodology

In this experiment, male C57BL/6 mice aged around 8-10 weeks were intraperitoneally injected with MPTP (20 mg/kg) twice a week (Monday and Thursday every week) for five weeks; test compounds A and B were intraperitoneally injected once daily (QD) for 6 consecutive weeks, and were given 30 min before MPTP on days of MPTP administration. Animals in control group were injected with an equal volume of normal saline. See Table 1 below for grouping. After 6 weeks, mice were euthanized and the dorsal striatum was separated after normal saline perfusion, weighed and preserved in a -80 °C freezer. The content of dopamine in dorsal striatal was determined by LC-MS.

**Table 1. Treatment of MPTP-induced mouse Parkinson's disease model with the combination or monotherapies**

| Grouping | Number of animals (n) | MPTP treatment | Test compound treatment |
|---|---|---|---|
| Group 1 (G1) | n=5 | 0 mg/kg, intraperitoneal injection, twice a week, for 5 weeks | 0 mg/kg, intraperitoneal injection, QD, for 6 weeks |
| Group 2 (G2) | n=12 | 20 mg/kg, intraperitoneal injection, twice a week, for 5 weeks | 0 mg/kg, intraperitoneal injection, QD, for 6 weeks |
| Group 3 (G3) | n=12 | 20 mg/kg, intraperitoneal injection, twice a week, for 5 weeks | Compound B, 15 mg/kg, intraperitoneal injection, QD, for 6 weeks |
| Group 4 (G4) | n=12 | 20 mg/kg, intraperitoneal injection, twice a week, for 5 weeks | Compound A, 0.05 mg/kg, intraperitoneal injection, QD, for 6 weeks |
| Group 5 (G5) | n=12 | 20 mg/kg, intraperitoneal injection, twice a week, for 5 weeks | Compound A, 0.1 mg/kg, intraperitoneal injection, QD, for 6 weeks |
| Group 6 (G6) | n=12 | 20 mg/kg, intraperitoneal injection, twice a week, for 5 weeks | Compound A, 0.15 mg/kg, intraperitoneal injection, QD, for 6 weeks |
| Group 7 (G7) | n=12 | 20 mg/kg, intraperitoneal injection, twice a week, for 5 weeks | Compound A, 0.2 mg/kg, intraperitoneal injection, QD, for 6 weeks |
| Group 8 (G8) | n=12 | 20 mg/kg, intraperitoneal injection, twice a week, for 5 weeks | Compound A, 0.5 mg/kg, intraperitoneal injection, QD, for 6 weeks |
| Group 9 (G9) | n=12 | 20 mg/kg, intraperitoneal injection, twice a week, for 5 weeks | Compound A (0.05 mg/kg, QD) + compound B (15 mg/kg, QD), intraperitoneal injection, for 6 weeks |
| Group 10 (G10) | n=12 | 20 mg/kg, intraperitoneal injection, twice a week, for 5 weeks | Compound A (0.1 mg/kg, QD) + compound B (15 mg/kg, QD), intraperitoneal injection, for 6 weeks |
| Group 11 (G11) | n=12 | 20 mg/kg, intraperitoneal injection, twice a week, for 5 weeks | Compound A (0.15 mg/kg, QD) + compound B (15 mg/kg, QD), intraperitoneal injection, for 6 weeks |
| Group 12 (G12) | n=12 | 20 mg/kg, intraperitoneal injection, twice a week, for 5 weeks | Compound A (0.2 mg/kg, QD) + compound B (15 mg/kg, QD), intraperitoneal injection, for 6 weeks |
| Group 13 (G13) | n=12 | 20 mg/kg, intraperitoneal injection, twice a week, for 5 weeks | Compound A (0.5 mg/kg, QD) + compound B (15 mg/kg, QD), intraperitoneal injection, for 6 weeks |

| | | | |
|---|---|---|---|
| Note: the administration volume was 10 mL/kg. On day one, the test compounds were first injected intraperitoneally and then MPTP was injected intraperitoneally 30 min later. For animals in groups 9 to 13, MPTP was intraperitoneally administered 30 min after administration of compounds A and B. | | | |

### (3) Results

The P values for comparisons of the above groups with the normal saline group G1, the modeling group G2 and the compound B group G3 are shown in Table 2 below (T-TEST unpaired t test, two-tailed):

**Table 2.**

| Group | | T-TEST-G1 (Normal saline) | T-TEST-G2 (Modeling group) | T-TEST-G3 B(15mg/kg) |
|---|---|---|---|---|
| Group 1 (G1) | Normal saline group | VS Saline | 0.00015867 | 0.004128274 |
| Group 2 (G2) | Modeling group | 0.00015867 | VS Model | 1.33956E-10 |
| Group 3 (G3) | Compound B (15 mg/kg) | 0.004128274 | 1.33956E-10 | VS B |
| Group 4 (G4) | Compound A (0.05 mg/kg) | 0.000108676 | 0.000560427 | 4.33604E-09 |
| Group 5 (G5) | Compound A (0.1 mg/kg) | 0.000414224 | 0.001230318 | 0.000124081 |
| Group 6 (G6) | Compound A (0.15 mg/kg) | 0.000181923 | 0.000448433 | 3.37881E-07 |
| Group 7 (G7) | Compound A (0.2 mg/kg) | 0.001307066 | 0.013642873 | 0.012684014 |
| Group 8 (G8) | Compound A (0.5 mg/kg) | 0.001764261 | 0.001936585 | 0.035856788 |
| Group 9 (G9) | Compound A (0.05 mg/kg) + Compound B (15 mg/kg) | 0.038747195 | 3.39672E-08 | 0.02117314 |
| Group 10 (G10) | Compound A (0.1 mg/kg) + Compound B (15 mg/kg) | 0.873635498 | 0.000230077 | 0.010381237 |
| Group 11 (G11) | Compound A (0.15 mg/kg) + Compound B (15 mg/kg) | 0.125276833 | 7.07842E-11 | 3.31108E-05 |
| Group 12 (G12) | Compound A (0.2 mg/kg) + Compound B (15 mg/kg) | 0.954019053 | 4.63272E-05 | 0.003097193 |
| Group 13 (G13) | Compound A (0.5 mg/kg) + Compound B (15 mg/kg) | 0.247059784 | 3.65199E-05 | 0.025978093 |

### (1) Comparison with normal saline group G1:

Compared with G1, groups G2, G4, G5 and G6 showed that P < 0.001, suggesting extremely significant differences in striatal dopamine concentration; compared with G1, groups G3, G7 and G8 showed that 0.001 < P < 0.01, suggesting significant differences; compared with G1, group G9 showed that 0.01 < P < 0.05, suggesting a difference; compared with G1, groups G10, G11, G12 and G13 showed that P > 0.05, suggesting no differences.

### (2) Comparison with modeling group G2:

Compared with G2, group G7 showed that 0.001 < P < 0.01, suggesting a significant difference in striatal dopamine concentration; compared with G2, group G8 showed that P > 0.05, suggesting no difference; compared with G2, group G13 showed that 0.01 < P < 0.05, suggesting a difference; compared with G2, the other groups showed that P < 0.001, suggesting extremely significant differences.

### (3) Comparison with compound B group G3:

Compared with G3, group G7 showed that P > 0.05, suggesting no difference in striatal dopamine concentration; compared with G3, groups G8, G9 and G13 showed that 0.01 < P < 0.05, suggesting significant differences; compared with G3, the other groups showed that P < 0.001, suggesting extremely significant differences.

The above results show that the dopamine levels in dorsal striatum in the modeling group G2 were significantly lower than those in the normal saline group G1; except for group G8, the dopamine levels in dorsal striatum in the compound A groups were significantly higher than those in the modeling group G2, while group G8 showed no significant difference; the dopamine levels in dorsal striatum in the compound B groups were significantly higher than those in the modeling group G2; the dopamine levels in dorsal striatum in the combination groups were significantly higher than those in the modeling group G2, and showed no significant difference from those in the normal saline group G1. This indicates that, after treated with the combination therapy, the dopamine levels significantly increased in dorsal striatum in MPTP-modeled mice; the dopamine levels of the compound A (0.1 mg/kg) + compound B (15 mg/kg) group G10 and the compound A (0.2 mg/kg) + compound B (15 mg/kg) group G12 demonstrated the least significant differences from the normal saline group G1 and the most significant differences from the modeling group G2, suggesting the best efficacies.

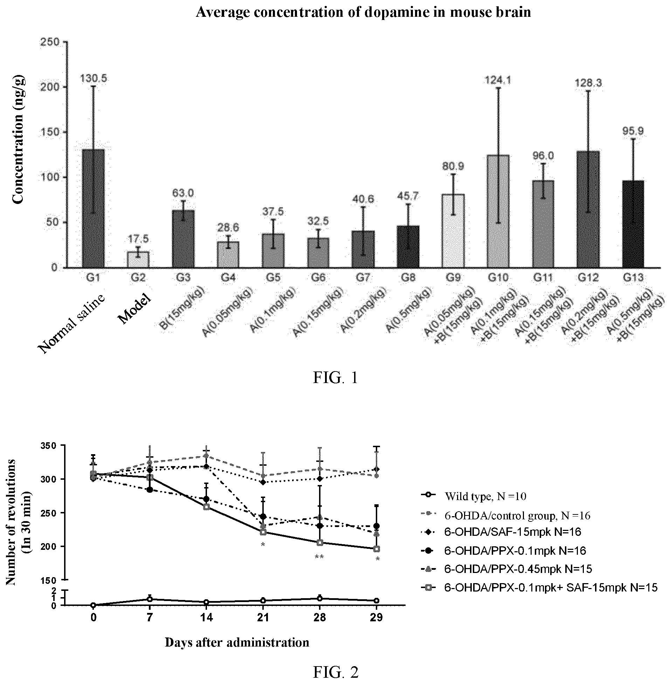

The corresponding results are shown in FIG. 1. As shown in the figure, pramipexole and safinamide both demonstrated improvement effects on dopamine reduction in this model, and the combination of the two drugs is significantly elevated the improvement effects. The pramipexole injection monotherapy showed dose-dependent effect to a certain extent; for the combination, although the dose dependence was irregular due to greater errors in several groups and an optimal combination cannot be determined, it can be seen that after the second dose group (G10), the efficacy was not improved significantly and the highest dose group even showed a significant reduction. This indicated that a good efficacy can be achieved at a low dose of pramipexole in the combination, which is consistent with the final purpose of reducing the dose of pramipexole so as to reduce risks of side effects and drug resistance while achieving similar or superior therapeutic effect. From this result, we can determine a range of the combination where pramipexole (0.05, 0.1, 0.15, 0.2 or 0.5 mg/kg) has a synergic effect with safinamide (15 mg/kg). Considering that the amount of pramipexole used is minimized without affecting the efficacy (and thus the side effects can be reduced), it is believed that the optimal effect of the experiment can be achieved when 0.1 mg/kg of pramipexole is combined with 15 mg/kg of safinamide, i.e., the dopamine concentration in the brain tissue of the animal model can be maximized.

### Example 2. In vivo characterization of the combination in a rat Parkinson's disease model with 6-OHDA (6-hydroxydopamine)-induced unilateral damage

### (1) Materials

150 Wistar rats, male, supplier: Beijing VR The animals were sent to Shanghai Ruizi Chemical Research Co., Ltd., and accommodated in the animal room at 2-3 mice/cage at 20-26 °C in a 12/12-h light/dark cycle (5:00 am to 5:00 pm in light).
Pramipexole hydrochloride: Manufacturer: Adamas; Purity: 99%; batch No.: P1448882
Safinamide mesylate: Manufacturer: Shanghai Pharmaceuticals Holding Co., Ltd.; Purity: 99.95%; batch No.: G059-S-2-151104
6-Hydroxydopamine: Manufacturer: Sigma Aldrich (Shanghai) Trading Co., Ltd.; batch No.: MKCD0817.

Other primary reagents and compounds are shown in Table 3 below:

**Table 3.**

| **Name** | **Manufacturer** | **Specifications** | **Batch No.** | **Date of expiration** |
|---|---|---|---|---|
| Sodium pentobarbitone | Merck | 25 g/bottle | NA | 2020.01.31 |
| Ascorbic acid | National Institutes for Food and Drug Control, PRC | 100 mg/vial | 100425-201504 | NA |
| Apomorphine | USP | 250mg | R08440 | NA |

### (2) Formulation

### 2.1 Formulation of 0.5 mg/kg Apo solution

2.1.1 4.44 mg of apomorphine (CF =1.17) was weighed accurately and added to 15.180 mL of sterile normal saline. The mixture was vortexed for 10 min in dark to obtain a clear and transparent solution. The solution was prepared right before use, and was kept in ice in use.

### 2.2 Formulation of 6-OHDA

2.2.1 0.02% (0.02 g/100 mL) ascorbic acid: 7.81 mg of ascorbic acid was weighed accurately and added to 39.05 mL of normal saline. The mixture was vortexed for 2 min to obtain a clear solution. The formulation process was conducted in dark and the solution was preserved at 4 °C.

2.2.1 5 mg/mL 6-OHDA (0.02% ascorbic acid): 0.663 mL of 0.02% ascorbic acid was added to the vial of 6-OHDA (5 mg package) (freebase = 98% × 5 mg/1.479 = 3.313 mg). The mixture was vortexed for 2 min to obtain a clear solution. A second vial was prepared using the same method. The 5 µg/µL 6-OHDA solution was subpackaged into 33 vials of 40-µL stock solution, which were preserved at -80 °C. The entire process was conducted in dark.

### 2.3 Formulation of 15 mg/kg (3 mg/mL) safinamide mesylate

2.3.1 359.83 mg of safinamide mesylate (CF = 1.318, 99.95% purity) was weighed accurately, and added to 90.959 mL of ultrapure water. The mixture was vortexed for 5 min in dark to obtain a clear and transparent solution, which was preserved at 4 degrees.

### 2.4 Formulation of 0.45 mg/kg (0.9 mg/mL) pramipexole hydrochloride

2.4.1 6.74 mg of pramipexole hydrochloride (CF = 1.430, 99% purity) was weighed accurately, and added to 51.844 mL of ultrapure water. The mixture was vortexed for 5 min in dark to obtain a clear and transparent solution, which was preserved at 4 degrees.

### 2.5 Formulation of 0.1 mg/kg (0.02 mg/mL) pramipexole hydrochloride

2.5.1 2.87 mg of pramipexole hydrochloride (CF = 1.430, 99% purity) was weighed accurately, and added to 99.350 mL of ultrapure water. The mixture was vortexed for 5 min in dark to obtain a clear and transparent solution, which was preserved at 4 degrees.

All formulations were conducted based on this standard and were diluted proportionally.

### (3) Procedures

3.1 The animals were pre-screened by an apomorphine-induced contralateral circling test before surgery.

3.1.1 On the day of testing, the animals were adapted in the operation room for more than 30 min.

3.1.2 The animals were injected intraperitoneally with APO (0.5 mg/kg, 2 mL/kg) 3 minutes before testing.

3.1.3 The animals were placed in a circular test box after dosing. Changes in animal behavior were observed and recorded by Ethovision video software, and the number of revolutions of each animal within 30 min was counted manually.

3.1.4 APO-induced circling behavior: The animal circled rightwards with the left hind limb as the fulcrum. One turn was counted as one revolution by taking the head of the rat as a reference. Animals with more than 180 revolutions within 30 min were qualified rat Parkinson's disease models.

3.1.5 After the test, the animals were returned to the homecage.

### 3.2 Preparation of 6-OHDA-induced rat Parkinson's disease model

3.2.1 140 animals were randomly selected, and intramuscularly injected with Zoletil at 30 mg/kg in combination with 3 mg/kg of xylazine hydrochloride for general anesthesia before the operation.

3.2.2 Rats were fixed on a stereotaxic apparatus. The skull epidermis was incised, the anterior and posterior fontanels were found, and the tooth bar was adjusted to keep the skull surface horizontal.

3.2.3 Taking the anterior fontanel as zero point, the coordinates were identified on the skull surface: AP: 0.5 mm; ML: -2.8 mm; DV: -4.5 mm.

3.2.4 A round hole slightly larger than the diameter of the micro injection needle was drilled at the coordinates by using a skull drill. The micro injection needle was adjusted to the surface of the dura mater and set as the zero point.

3.2.5 The light was turned off and a yellow light source was turned on. A volume of 6-OHDA slightly greater than the injection amount was taken in.

3.2.6 The micro injection needle was slowly screwed down to a designated depth in the brain of the rat within about 5 min.

3.2.7 The injection of 6-OHDA was started 2 min after the needle reached the designated depth. The injection amount was 4 µL (5 µg/µL), with the total amount of 6-OHDA being 20 µg. The injection speed was 1 µL/min.

3.2.8 After the injection was finished, the needle was left for 5 min. The micro injection needle was then slowly screwed out in about 5 min.

3.2.9 After the wound was sutured and disinfected, the rat was returned to the homecage. The rats were kept warm and water was given before the rats regained their consciousness. The wound, respiration and pain frequency, and excretion were observed.

3.2.10 The surgical instruments were cleaned and the micro injector was rinsed with normal saline to prevent clogging everyday after operation.

3.2.11 The animals were subjected to a 3-day postoperative care.

### 3.3 Qualified animal models were screened on Days 7, 14 and 21 after operation by an apomorphine-induced contralateral circling test in the PD rats.

3.3.1 On the day of testing, the animals were adapted in the operation room for more than 30 min.

3.3.2 The animals were injected intraperitoneally with APO (0.5 mg/kg, 2 mL/kg) 3 minutes before testing.

3.3.3 The animals were placed in a circular test box after dosing. Changes in animal behavior were observed and recorded by Ethovision video software, and the number of revolutions of each animal within 30 min was counted manually.

3.3.4 APO-induced circling behavior: The animal circled rightwards with the left hind limb as the fulcrum. One turn was counted as one revolution by taking the head of the rat as a reference. Animals with more than 180 revolutions within 30 min were qualified rat Parkinson's disease models.

3.3.5 After the test, the animals were returned to the homecage.

### 3.4 Therapeutic effect of continuous treatment on 6-OHDA-induced rat Parkinson's disease model

3.4.1 Apomorphine-induced contralateral circling test was performed on day 21 after operation, 78 animals with >180 revolutions in 30 min and 10 naive animals, for a total of 88 animals, were selected for administration and testing.

3.4.2 Qualified rats were assigned to various groups based on the number of revolutions on day 21 after operation to ensure similar levels of lesions among the groups before treatment.

3.4.3 After grouping, the treatment was given on day 22 after operation. The grouping is shown in Table 4 below.

**Table 4.**

| **Grou P** | **Model** | **Treatment** | **Number of animals** | **Route of administration** | **Frequency of administration** |
|---|---|---|---|---|---|
| G1 | Wild type | Normal saline | 10 | P.o. | QD×28 |
| G2 | 6-OHDA-induced unilateral damage PD model (Male wistar rat) | Normal saline | 16 (included) | P.o. | QD×28 |
| G3 | | Safinamide SAF (15 mpk) | 16 (included) | P.o. | QD×28 |
| G4 | | Low-dose pramipexole PPX (0.1 mpk) | 16 (included) | P.o. | QD×28 |
| G5 | | Combination (PPX 0.1 mpk/SAF 15 mpk) | 15 (included) | P.o. | QD×28 |
| G6 | | High-dose pramipexole PPX (0.45 mg/kg) | 15 (included) | P.o. | QD×28 |

In the table, PPX denotes pramipexole, and SAF denotes safinamide.

3.4.4 Apomorphine-induced circling test was performed prior to dosing on days 7, 14, 21 and 28.

3.4.5 The rats were subjected to the apomorphine-induced circling test the day after the last dose.

### (4) Results

Results of the apomorphine-induced circling test pre-dose, on days 7, 14, 21 and 28 and the next day of the last dose are shown in FIG. 2.

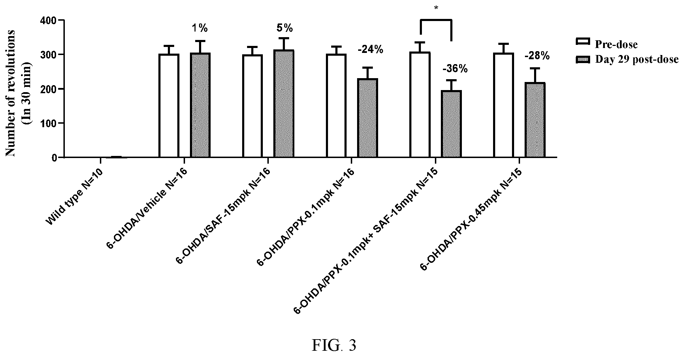

Compared with the control group (vehicle group), the combination group G5 (PPX 0.1 mpk/SAF 15 mpk) showed improved movement function of a rat PD model with timeliness and significance after 21 days of treatment, and a better efficacy in improving the movement function in the rat PD model than two monotherapy groups (G3 and G4); the combination also imparts neuroprotective activity and thus can reduce the normal sensitivity of the dopamine system to dopamine agonists, reducing or avoiding fluctuations in symptoms. After 29 days of treatment, the combination group G5 showed a 36% reduction in the number of revolutions in the rat PD model compared with that before treatment, while groups G3 and G4 showed a 5% elevation and a 24% reduction, respectively (see FIG. 3).

It can also be seen from the above data that the composition of this example can restore or equilibrate the potential inequilibrium response to the dopaminergic increase treatment caused by dopamine agonist alone; the composition reduces the amount of pramipexole to reduce the risks of side effects and drug resistance, while achieving the same or better therapeutic effect.

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A pharmaceutical composition comprising pramipexole and safinamide, wherein a mass ratio of the pramipexole and the safinamide is1:300 to 1:30.

2. The pharmaceutical composition according to claim 1, wherein the mass ratio of the pramipexole and the safinamide is 1:200 to 1:50, preferably 1:150 to 1:75, for example, 1:55, 1:60, 1:65, 1:70, 1:75, 1:80, 1:85, 1:90, 1:95, 1:100, 1:110, 1:120, 1:130, 1:140, 1:150, 1:160, 1:170, 1:180 or 1:190.

3. The pharmaceutical composition according to claim 1 or 2, wherein an amount of the pramipexole is 0.01 to 5 mg; and/or an amount of the safinamide is 0.3 to 1500 mg;
preferably, the amount of the pramipexole is 0.05 to 0.5 mg; and/or the amount of the safinamide is 7.5 to 100 mg.

4. The pharmaceutical composition according to claim 3, wherein the amount of the pramipexole is 0.02, 0.04, 0.05, 0.06, 0.08, 0.1, 0.15, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 3 or 4 mg, preferably, 0.1, 0.15 or 0.2 mg; and/or the amount of the safinamide is 0.4, 0.5, 0.6, 0.7, 0.8, 0.9, 1, 1.1, 1.2, 1.3, 1.4, 1.5, 1.6, 1.7, 1.8, 1.9, 2, 3, 4, 5, 6, 7, 7.5, 8, 9, 10, 12, 14, 15, 16, 18, 20, 30, 40, 50, 60, 70, 80, 90, 100, 110, 120, 130, 140, 150, 200, 300, 400, 500, 600, 700, 800, 900, 1000, 1100, 1200, 1300.0 or 1400 mg, preferably 10.0, 15, 20 or 50 mg.

5. The pharmaceutical composition according to claim 4, wherein the pharmaceutical composition comprises 0.05 mg of pramipexole and 7.5 mg of safinamide, 0.05 mg of pramipexole and 10 mg of safinamide, 0.05 mg of pramipexole and 15 mg of safinamide, 0.1 mg of pramipexole and 15 mg of safinamide, 0.15 mg of pramipexole and 15 mg of safinamide, 0.2 mg of pramipexole and 15 mg of safinamide, 0.5 mg of pramipexole and 15 mg of safinamide, 0.6 mg of pramipexole and 60 mg of safinamide, 1.2 mg of pramipexole and 120 mg of safinamide, 0.3 mg of pramipexole and 30 mg of safinamide, or 0.2 mg of pramipexole and 20 mg of safinamide.

6. The pharmaceutical composition according to any one of claims 1 to 5, the pharmaceutical composition further comprises at least one pharmaceutically acceptable carrier.

7. A kit of parts comprising a kit A and a kit B, wherein the kit A comprises pramipexole and the kit B comprises safinamide, or the kit A comprises pramipexole and safinamide and the kit B comprises another drug; wherein a mass ratio of pramipexole and safinamide is 1:300 to 1:30, preferably 1:200 to 1:50, more preferably 150 to 1:75, for example, 1:55, 1:60, 1:65, 1:70, 1:75, 1:80, 1:85, 1:90, 1:95, 1:100, 1:110, 1:120, 1:130, 1:140, 1:150, 1:160, 1:170, 1:180 or 1:190.

8. The kit of parts according to claim 7, wherein an amount of the pramipexole is 0.01 to 5 mg, preferably 0.05 to 0.5 mg; and/or an amount of the safinamide is 0.3 to 1500 mg, preferably 7.5 to 100 mg;
the amount of the pramipexole is, for example, 0.02, 0.04, 0.05, 0.06, 0.08, 0.1, 0.15, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 3 or 4 mg, preferably, 0.1, 0.15 or 0.2 mg; and/or the amount of the safinamide is, for example, 0.4, 0.5, 0.6, 0.7, 0.8, 0.9, 1, 1.1, 1.2, 1.3, 1.4, 1.5, 1.6, 1.7, 1.8, 1.9, 2, 3, 4, 5, 6, 7, 7.5, 8, 9, 10, 12, 14, 15, 16, 18, 20, 30, 40, 50, 60, 70, 80, 90, 100, 110, 120, 130, 140, 150, 200, 300, 400, 500, 600, 700, 800, 900, 1000, 1100, 1200, 1300 or 1400 mg, preferably 10, 15, 20 or 50 mg;
more preferably, the kit of parts comprises 0.05 mg of pramipexole and 7.5 mg of safinamide, 0.05 mg of pramipexole and 10 mg of safinamide, 0.05 mg of pramipexole and 15 mg of safinamide, 0.1 mg of pramipexole and 15 mg of safinamide, 0.15 mg of pramipexole and 15 mg of safinamide, 0.2 mg of pramipexole and 15 mg of safinamide, 0.5 mg of pramipexole and 15 mg of safinamide, 0.6 mg of pramipexole and 60 mg of safinamide, 1.2 mg of pramipexole and 120 mg of safinamide, 0.3 mg of pramipexole and 30 mg of safinamide, or 0.2 mg of pramipexole and 20 mg of safinamide.

9. Use of the pharmaceutical composition according to any one of claims 1 to 6 or the kit of parts according to claim 7 or 8 in preparing a medicament for treating Parkinson's disease, for improving movement function and/or for increasing dopamine concentration.

10. Use of the pharmaceutical composition according to any one of claims 1 to 6 or the kit of parts according to claim 7 or 8 in treating Parkinson's disease, in improving movement function and/or in increasing dopamine concentration.

11. A method of treating Parkinson's disease comprising: administering to a patient in need thereof the pharmaceutical composition according to any one of claims 1 to 6 or the kit of parts according to claim 7 or 8.
